# EUROPÄISCHE PATENTSCHRIFT

(11) **EP 0 317 591 B1**
(45) Veröffentlichungstag und Bekanntmachung des Hinweises auf die Patenterteilung: **02.08.1995**
(21) Anmeldenummer: 88904523.3
(22) Anmeldetag: 19.05.1988
(51) Int. Cl.: A61B 5/103

(54) **VERFAHREN UND VORRICHTUNG ZUR HERSTELLUNG VON EINLAGEN ODER DERGLEICHEN**
PROCESS AND DEVICE FOR MANUFACTURING INTERLININGS AND SIMILAR
PROCEDE ET DISPOSITIF POUR LA FABRICATION DE GARNITURES OU SIMILAIRE

(30) Priorität: 21.05.1987 DE 3717126
(43) Veröffentlichungstag der Anmeldung: 31.05.1989
(73) Patentinhaber: Seitz, Peter, D-81675 München (DE)
(72) Erfinder: Seitz, Peter, D-81675 München (DE)
(74) Vertreter: Bohnenberger, Johannes, Dr.
(86) Internationale Anmeldenummer: EP8800444
(87) Internationale Veröffentlichungsnummer: WO8809147

(56) Entgegenhaltungen:
- DE-A- 2 821 247
- DE-A- 3 214 306
- FR-A- 2 331 934
- FR-A- 2 513 508
- NL-A- 8 001 419
- US-A- 3 696 456
- US-A- 4 195 643
- Computer Systems, volume 6, No. 10, October 1986, Bromley (GB) "Shape a leg" see pages 15,16; see pages 15,16, left hand column, line 8 - middle column, line 22; figures 1,2

## Beschreibung

Die Erfindung betrifft ein Verfahren zur Herstellung von Einlagen.

Hierbei ist der Begriff "Einlagen" als Oberbegriff für technische Gegenstände zu verstehen, die mit Körperteilen des Menschen in kraft- und/oder formschlüssige Verbindung gebracht werden. Ein typisches Beispiel sind othopädische Einlagen oder Fußbetten, die auf den Fuß eines Patienten, natürlich auch eines gesunden Menschen anzupassen sind. Ebenso dreht es sich um Sitz- oder Liegemöbel, die den entsprechenden Körperteilen des Benutzers anzupassen sind. Gleichfalls sind Prothesen oder Orthesen hierunter zu verstehen, die an einen Gliedstumpf anzupassen sind. Weiterhin dreht es sich bei der Erfindung auch um die Anpassung von Zahnprothesen (Vollprothesen ebenso wie einzelne Zähne oder Kronen),die insbesondere unter Berücksichtigung der Bewegungsgeometrie des Kauapparates an die gegenüberstehenden (natürlichen oder ebenfalls künstlichen) Zähne anzupassen sind.

Bei der Herstellung von orthopädischen Einlagen (ebenso z.B. von Prothesen oder Korsetts) geht man gegenwärtig davon aus, daß man eine (räumliche) Vermessung der anzupassenden bzw. zu korrigierenden Formen vornimmt. Diese Vermessung kann über Abdrücke in aushärtenden Massen oder über eine direkte (optische) Vermessung stattfinden. In jedem Fall wird also praktisch ausschließlich von räumlichen Konturen ausgegangen, wobei darüber hinaus im allgemeinen der unbelastete Zustand des jeweiligen Körperteils vorliegt.

Es hat sich nun gezeigt, daß Einlagen, Prothesen oder auch Sitz- oder Liegemöbel, die basierend auf diesen Vermessungen gefertigt werden, vom Benutzer oftmals als unangenehm, störend usw. empfunden werden. Darüber hinaus sind die Korrekturresultate nicht optimal. Schließlich besteht insbesondere bei Einlagen/Fußbetten das Problem, daß der Orthopädieschuhmachermeister in vielen Punkten "nach Gefühl" arbeitet, die von ihm hergestellten Einlagen also nicht ohne weiteres aufgrund reiner, objektiver Meßwerte herstellbar sind. Beim Herstellen von Zahnprothesen wird noch ungenauer vorgegangen, wobei wiederum lediglich eine direkte Vermessung der räumlichen Konturen stattfindet und zwar über Abdrücke in Kunststoffnassen und nachträgliches Abschleifen von "Druckstellen", die sich beim Beißen auf eingefärbtes Papier auf der Zahnprothese abzeichnen.

Aus der US-A-369 645 6 ist es bekannt, daß man einen Schuhleisten unter direkter Form-Abtastung eines Fußes automatisiert herstellen kann. Es hat sich jedoch gezeigt, daß ein Schuh, der unter Zuhilfenahme eines solchen Leistens hergestellt wurde, keine optimalen Eigenschaften in orthopädischer Sicht aufweist. Ähnliche Gedanken in Bezug auf die Anfertigung einer Prothese sind der Zeitschrift "Computer Systems", Band 6, Nr. 10, Oktober 1986, Bromley (GB); "Shape a Leg" oder in Bezug auf die Anfertigung einer Sohlenform aus der DE-A-282 124 7 bekannt. In beiden Fällen ergeben sich die oben angeführten Nachteile.

Aus der FR-A-251 350 8 ist es bekannt, daß man ein Druckkraft-Verteilungsmuster unter dem Fuß eines Probanden messen kann, wenn dieser auf einer Meßplattform steht oder über diese geht. In der Druckschrift wird weiterhin angegeben, man könne anhand eines Druckkraft-Verteilungsmusters, das beim Gehen eines Patienten über eine Meßplattform angezeigt wird, Defekte erkennen und eine Korrektur über ein orthopädisches Element vornehmen. Nach der Korrektur soll das Ergebnis wieder überprüft werden.

Es hat sich jedoch gezeigt, daß diese rein empirische Vorgehensweise keine Verbesserung bei der Herstellung von Einlagen erzielen läßt.

Der Erfindung liegt die Aufgabe zugrunde, ein Verfahren sowie eine Vorrichtung zur Durchführung des Verfahrens aufzuzeigen, mittels derer in einfacher Weise verbesserte orthopädische Wirkungen erzielbar sind.

Diese Aufgabe wird alternativ durch die Verfahren nach einem der Ansprüche 1 bis 3 gelöst.

Insbesondere dreht es sich also bei der vorliegenden Erfindung darum, daß die Kraftverteilung bzw. das zwei- oder dreidimensionale Muster der auftretenden Kräfte zur Erstellung der Einlagen, Sitzmöbelflächen usw. herangezogen wird, und zwar derart, daß eine direkte Steuerung der die Teile fertigenden Maschine ermöglicht wird. Unter direkte Steuerung ist hier auch der Fall als verschlechterte Ausführungsform zu verstehen, bei dem eine konforme Abbildung des zu erstellenden Gegenstandes vom Rechner produziert wird und dann in (allerdings etwas mühsamer) Arbeit nach dieser konformen Abbildung der Gegenstand gefertigt wird. Auch der Ausdruck von entsprechenden Sätzen von Meßwerten ist hier umfaßt. In allen Fällen ergibt sich jedoch eine einwandfreie Reproduzierbarkeit des Verfahrens, was die Qualität des entstehenden Produktes erhöht und gleichzeitig die Kosten senkt.

Die Herstellung von orthopädischen Einlagen kann hierbei so erfolgen, daß die vom Rechner erzeugten Steuerdaten eine numerisch gesteuerte Maschine zur Herstellung der Einlage selbst steuern. Dies kann z.B. durch Ausfräsen, durch entsprechendes Heiß- oder Kaltverformen hinsichtlich der geeometrischen Abmessungen geschehen, wobei durch die entsprechende Formung eine Annäherung an das "Ideal"-Druckbild erzeugt bzw. überhohe Drücke an bestimmten Stellen (z.B. durch Einsenkungen in der Einlage) aufgefangen bzw. kompensiert werden. Wenn es sich um die Anpassung von Prothesen dreht, so kann durch die Auswertung des Druckbildes eine optimale Anpassung des Gebisses bzw. der Kauflächen an die Bewegungsgeometrie des Kauapparates erzielt werden, was bisher nur durch sehr komplizierte räumliche Vermessung möglich ist. Insbesondere werden in diesem Falle auch dreidimensionale Meßwerte, also Scherkräfte berücksichtigt, die an den Kauflächen wirken.

Eine weitere, im Rahmen der Erfindung befindliche Möglichkeit besteht darin, lediglich oder auch zusätzlich die Härte bzw. Steifigkeit des Materials, aus dem die Einlagen oder Sitzflächen usw. gefertigt werden, numerisch gesteuert zu bestimmen. Hierbei ist es möglich, z.B. die Zusammensetzung eines Kunststoffes, aus dem die Einlagen (oder die Matratze) gefertigt werden bzw. mit dem ein Stuhl-Rohling beschichtet wird, ortsabhängig einzustellen, den Aufschäumgrad ortsabhängig zu bestimmen, die Aushärtung eines aushärtbaren Kunststoffes ortsabhängig zu steuern, die Dichte des Materials (z.B. durch Bohrungen oder durch Pressen) ortsabhängig einzustellen usw..

Bei einer weiteren, bevorzugten Ausführungsform der Erfindung wird zusätzlich zur Kraftverteilung zumindest in den Bereichen, in denen der Fuß bei Belastung nicht auf der Meßplattform aufliegt, die Entfernung der nicht aufliegenden Fußsohlenteile zur Meßplattform bestimmt und somit zusätzliche Information zur Ausgestaltung der Einlage im Sinne einer Kompensation von "Fehlformen" gewonnen und der die Einlagen herstellenden Maschine mitgeteilt.

Wenn man keine Einlagen fertigen, sondern z.B. einen Stuhl oder einen Kraftfahrzeugsitz aufbauen will, so geht man vorteilhafterweise nicht von einer flachen Meßplattform aus, sondern von einem "Normstuhl" bzw. einem Rohling, dessen dem Körper gegenüberliegende Flächen mit entsprechenden Kraft-Meßaufnehmern (vorzugsweise zusätzlich Distanzaufnehmern) ausgerüstet wird. Sitzt ein Patient auf einem solchen Rohling, so ergibt sich bei der Messung ein "Fehlermuster", aufgrund dessen (unter Berücksichtigung der Formen des Rohlings) dann die numerisch gesteuerte Maschine das tatsächlich dem Probanden anzupassende Sitzpolster fertigen kann. Gleiches gilt selbstverständlich auch für die Anpassung von Prothesen. Auch hier dreht es sich also im wesentlichen um die Messung von Kräften, deren Vergleich mit einem Satz von Sollwerten und die nach Errechnung von entsprechenden Steuersignalen selbsttätige und damit exakt reproduzierbare Fertigung von Sitzmöbeln/Prothesen/Orthesen über numerisch gesteuerte Maschinen.

Die Eingabe der Steuerdaten in die Maschine zur Herstellung der Einlagen usw. muß nicht on line erfolgen, sondern kann auch von Hand durchgeführt werden, wie dies eingangs beschrieben wurde. Ebenso ist es im Prinzip möglich, anstelle von numerisch gesteuerten Maschinen z.B. Schablonen zu fertigen, die zum Nacharbeiten von Hand dienen. Diese verschlechterte Ausführungsform des Verfahrens fällt selbstverständlich auch unter den Erfindungsgedanken.

Bevorzugte Ausführungsformen der Erfindung sind in den Unteransprüchen beschrieben.

Zum besseren Verständnis werden im folgenden bevorzugte AuSführungsformen der Erfindung anhand von Abbildungen näher erläutert. Hierbei zeigen:
- Fig. 1: einen schematisierten Aufbau einer Vorrichtung zur Durchführung des erfindungsgemäßen Verfahrens;
- Fig. 2: eine Seitenansicht einer Meßplattform mit aufgelegtem Fuß;
- Fig. 3: eine Prinzipdarstellung des in Fig. 2 mit III bezifferten Bereiches;
- Fig. 4: eine weitere bevorzugte Ausführungsform einer Distanz-Meßvorrichtung; und
- Fig. 5: eine Detaildarstellung eines einzelnen Distanz-Meßaufnehmers aus Fig. 4.

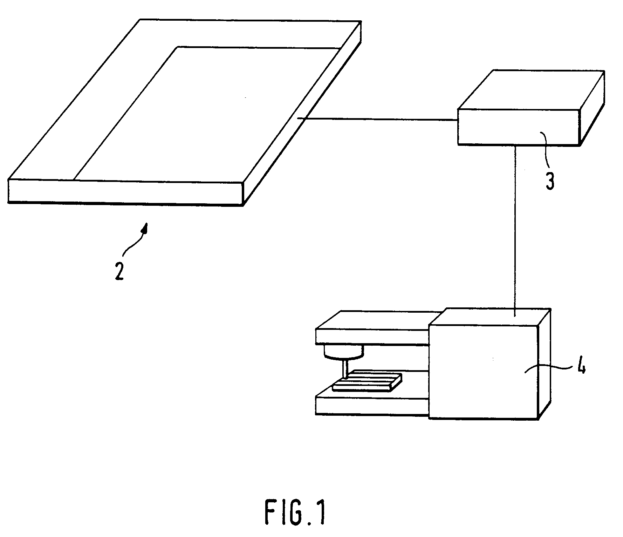

Wie in Fig. 1 gezeigt, ist eine Meßplattform 2 mit einem Rechner 3 verbunden, dessen Ausgang eine numerisch gesteuerte Fräse (Bohrmaschine usw.) steuert. Diese Vorrichtung zur Herstellung der Einlagen (Prothesen usw.) ist mit der Bezugsziffer 4 versehen.

Man führt nicht nur rein statische Messungen durch, sondern läßt den Patienten mehrmals über die Meßplattform 2 laufen und mittelt über das entstehende Druckverteilungsmuster. Hierbei wird im Rechner 3 eine Zuordnung des momentan gemessenen "Druckbildes" zum zuvor gemessenen Druckbild hinsichtlich der Lage in der Meßebene vorgenommen. Durch diese Mittelung ist eine wesentliche Verbesserung des Einlagensitzes möglich, da das tatsächliche Druckmuster nicht nur mit erhöhter Genauigkeit, sondern auch unter Ausschluß verschiedener verfälschender Einflüsse (verkrampfte Körperhaltung usw.) herleitbar ist. Weiterhin wird eine "dynamische" Messung vorgenommen, die überraschenderweise wesentlich mehr über die tatsächlichen Fehler im Druckbild (gegenüber einem "normalen" Druckbild) aussagt, als die rein statische Messung. Als Meßplattform kann u.a. das unter dem Namen "EMED" vertriebene System dienen, über das z.B. in "Arzt heute", Seite 3 (16. Oktober 1985) berichtet wird. Andere Arten von ortsauflösenden Meßplattformen oder Meßflächen können ebenfalls verwendet werden.

Anstelle einer starren Plattform 2 kann (wie auch bei der Fertigung von Prothesen/Korsetts) eine flexible Meßmatte Verwendung finden, die (in Form einer Einlegesohle ausgeschnitten) in einen Schuh einlegbar ist. Das beim Gehen (Laufen, Stehen) des Probanden meßbare Druckverteilungsbild umfaßt dann auch den Einfluß des Schuhs selbst, so daß eine zusätzliche Anpassung einer Einlage oder eines Fußbettes auch an den Schuh erfolgen kann. Insbesondere zur Herstellung von Fußbetten (bei vorgegebenen Schuhen), ist dies von Vorteil. Weiterhin ist es möglich, zusätzlich noch eine "Norm-Einlage" zwischen Schuh und Meßeinlage vorzusehen, die dann anhand der gewonnenen Meßdaten korrigierbar und somit an Fuß und Schuh gleichzeitig anpaßbar ist.

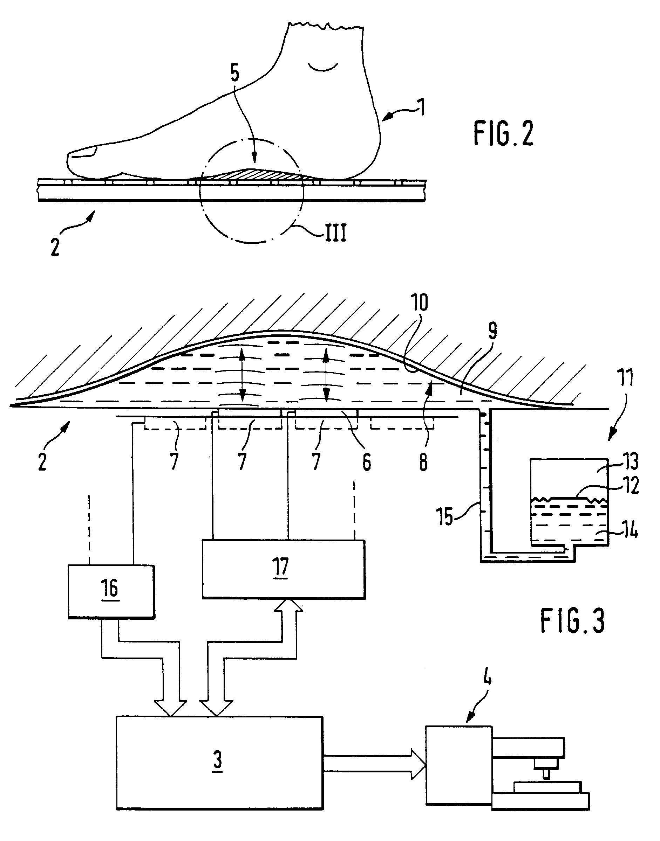

In Fig. 2 ist ein auf eine Meßplattform 2 aufgesetzter Fuß 1 in der Seitenansicht dargestellt. Auf der Meßplattform 2 sind weiterhin die Meßwertaufnehmer, z.B. kapazitive Aufnehmer, schematisiert dargestellt. Aus dieser Abbildung geht hervor, daß im Bereich zwischen Ferse und Vorderfuß ein Bereich 5 existiert, in welchem der Fuß (abgesehen von einem extremen Plattfuß) auf der Meßplattform 2 bzw. auf deren Oberfläche aufsitzt. Dieser Bereich III wird nun vorzugsweise hinsichtlich seiner geometrischen Abmessungen zusätzlich zur Druckverteilungsmessung ausgemessen, um die Einlage (Prothese, Korsett) entsprechend fertigen zu können. Eine bevorzugte Ausführungsform einer Anordnung zur Ausmessung derartige Räume ist in Fig. 3 gezeigt.

Hier ist dicht zur Oberfläche der Meßplattform 2 eine Folie 10 aufgebracht, die mit der Oberfläche der Meßplattform 2 einen Hohlraum bilden kann, welcher über eine Leitung 15 mit einer Flüssigkeit 9 aufgefüllt werden kann. Die Leitung 15 steht hierfür mit einer druckerzeugenden Einheit 11 in Verbindung, die bei dem in Fig. 3 gezeigten Beispiel einen mit Druckgas gefüllten Raum 13 umfaßt, der über eine Membran 12 von einem Vorratsraum 14 für Flüssigkeit 9 getrennt ist. Diese Anordnung wirkt bei entsprechend großer Ausbildung des Volumens so, daß ein von den geometrischen Abmessungen weitgehend unabhängiger Druck in der Flüssigkeit 9 herrscht, so daß die Folie 10 mit konstanter Kraft auf die Fußsohle drückt.

Auf der Oberfläche der Meßplattform 2 bzw. im wesentlichen direkt auf den Meßfühlern 7 sind Piezowandler 6 angeordnet, welche über eine Ansteuer- und Auswertschaltung 17 mit dem Rechner 3 verbunden sind. Die Piezowandler 6 senden (gepulste) Ultraschallsignale aus (Pfeile 1 Fig. 3), welche von der Folienoberfläche 10 reflektiert und von denselben (gegebenenfalls von weiteren) Piezowandlern empfangen und wieder an die Auswertschaltung 17 weitergegeben werden. Aus den Laufzeitdifferenzen ist der Abstand der Folie 10 und damit der Fußsohle im nicht aufliegenden Bereich meßbar. Vorzugsweise sind die Meßwandler 6 als Piezofolie ausgebildet, die auf beiden Seiten über matrixförmig angeordnete Leiterbahnen kontaktiert werden. Dadurch ist ein gesondertes Ansteuern und/oder Abfragen der Wandler möglich, um ein dreidimensionales Muster zu erzielen. Weiterhin ist es von Vorteil, wenn man mehrere Piezowandler 6 zur Erzielung einer Richtcharakteristik zusammenfaßt, was die Ortsauflösung dieser Meßanordnung weiter verbessert. Durch die Flüssigkeitsfüllung sind sehr hohe Frequenzen und damit eine sehr hohe Auflösung möglich, die Empfindlichkeit des Systems steigt aufgrund der geringen Dämpfung in der Flüssigkeit.

Bei einer weiteren, hier nicht gezeigten bevorzugten Ausführungsform der Erfindung sind anstelle von Piezowandlern 6 induktive oder kapazitive Meßaufnehmer vorgesehen, wobei dann die Folie 10 vorzugsweise metallbedampft ist.

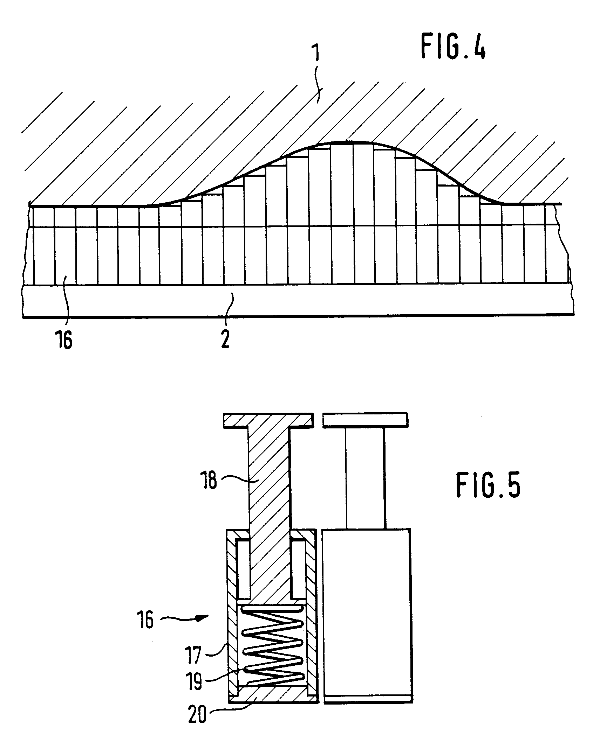

Bei der in Fig. 4 gezeigten Ausführungsform wird der Abstand zwischen der Meßplattform 2 und dem Fuß 1 über eine Federstempelanordnung gemessen, die auf einer starren Plattform 2 und deren Meßfläche gleichmäßig bedeckend nebeneinanderstehend angebracht sind. Jeder Federstempel 16 umfaßt ein Gehäuse 17, in dem teleskopisch ausfahrbar ein Stempel 18 sitzt. Jeder Stempel 18 wird über eine Feder 19 von der Plattform 2 fortgedrückt, wobei sein Auflager von einem Deckel 20 im Gehäuse 17 gebildet ist. Die Stärke der Federn 19 ist hierbei so gering bemessen, daß dort, wo der Fuß beim Stehen auf einer flachen, harten Unterlage aufliegt, die Federn 19 vollständig zusammengedrückt sind. Nur in den Bereichen, in denen der Fuß aufgrund seiner Form gegenüber einer flachen, harten Plattform beabstandet ist, sind die Stempel 18 ausgefahren. Hierbei übt jeder Stempel 18 über die Feder 19 und den Boden 20 eine Kraft auf die Meßplattform 2 aus, deren Amplitude umgekehrt proportional zur Längenausdehnung der Feder 19 und somit proportional zum Abstand des entsprechenden Fuß-Abschnittes gegenüber der durch die vollständig eingedrückten Stempel definierten Fläche ist. Über die Messung dieser Kräfte können somit gleichzeitig die Distanzen bestimmt werden.

Die in den Fig. 2 bis 5 gezeigten Anordnungen können auch zur optimalen Gestaltung von Liegemöbeln dienen. Bei einer bevorzugten Ausführungsform der Erfindung, die in den Abbildungen nicht dargestellt ist, geht man jedoch zur Herstellung von Liege- oder Sitzmöbeln von einem Rohling aus, dessen Form Durchschnittswerten angepaßt ist. Derartige Rohlinge sind dann mit einer Anordnung von Druckkraftaufnehmern 7 versehen. Für die Herstellung von Prothesen gilt entsprechendes.

Zur Herstellung von Zahnprothesen eignet sich insbesondere eine flexible Meßanordnung (wie sie z.B. auch als Einlegesohle wie oben beschrieben Verwendung finden kann), wobei hier auf eine geringe Dicke zu achten ist, damit die Bewegungsgeometrie des Kauapparates durch die Meßanordnung nicht in unzulässiger Weise verfälscht wird.

## Patentansprüche

1. Verfahren zur Herstellung von Einlagen oder dergleichen, wobei ein Patient mehrmals in einer definierten Bewegung mit dem anzupassenden Körperteil (Fuß, Gesäß/Rücken, Zähne) auf/über eine(r) elektronische(n) Meßanordnung zur zwei- oder dreidimensionalen Messung der auf die Meßanordnung wirkenden Kräfte schreitet/sitzt/liegt/beißt, die elektronische Meßanordnung in einer dynamischen Messung Ausgangssignale entsprechend einem über eine Vielzahl von Messungen gemittelten Druckkraft-Verteilungsmuster unter Zuordnung eines momentan gemessenen Druckkraft-Verteilungsmusters zum zuvor gemessenen Druckkraft-Verteilungsmuster hinsichtlich der Lage in der Meßebene erzeugt, die einem Rechner zugeleitet und mit einem gespeicherten Soll-Druckkraft-Verteilungsmuster verglichen werden, die Unterschiede zwischen den Druckkraft-Verteilungsmustern in Steuersignale zum Steuern einer Vorrichtung zum Herstellen von Einlagen (Sitzmöbelflächen, Liegemöbelflächen, Zahnprothesen) derart hinsichtlich der geometrischen Abmessungen der Einlagen (Sitzmöbelflächen, Liegemöbelflächen, Zahnprothesen) umgesetzt werden, daß sich bei Benutzung der hergestellten Einlagen oder dergleichen das Soll-Druckkraft-Verteilungsmuster ergibt.

2. Verfahren zur Herstellung von Einlagen oder dergleichen, wobei ein Patient mehrmals in einer definierten Bewegung mit dem anzupassenden Körperteil (Fuß, Gesäß/Rücken, Zähne) auf/über eine(r) elektronische(n) Meßanordnung zur zwei- oder dreidimensionalen Messung der auf die Meßanordnung wirkenden Kräfte schreitet/sitzt/liegt/beißt, die elektronische Meßanordnung in einer dynamischen Messung Ausgangssignale entsprechend einem über eine Vielzahl von Messungen gemittelten Druckkraft-Verteilungsmuster unter Zuordnung eines momentan gemessenen Druckkraft-Verteilungsmusters zum zuvor gemessenen Druckkraft-Verteilungsmuster hinsichtlich der Lage in der Meßebene erzeugt, die einem Rechner zugeleitet und mit einem gespeicherten Soll-Druckkraft-Verteilungsmuster verglichen werden, die Unterschiede zwischen den Druckkraft-Verteilungsmustern in Steuersignale zum Steuern einer Vorrichtung zum Herstellen von Einlagen (Sitzmöbelflächen, Liegemöbelflächen, Zahnprothesen) derart hinsichtlich der Härte oder Steifigkeit des Materials für die Einlagen (Sitzmöbelflächen, Liegemöbelflächen, Zahnprothesen) umgesetzt werden, daß sich bei Benutzung der hergestellten Einlagen oder dergleichen das Soll-Druckkraft-Verteilungsmuster ergibt.

3. Verfahren zur Herstellung von Einlagen, wobei eine flexible Meßmatte als Teil einer elektronischen Meßanordnung in einen Schuh eingelegt wird und das beim Gehen oder Laufen des Patienten auf die Meßmatte wirkende Druckkraft-Verteilungsmuster über eine Vielzahl von Messungen gemittelt und einem Rechner zugeleitet wird, wobei die Mittelung unter Zuordnung eines momentan gemessenen Druckkraft-Verteilungsmusters zum zuvor gemessenen Druckkraft-Verteilungsmuster hinsichtlich der Lage in der Meßebene erzeugt und mit einem gespeicherten Soll-Druckkraft-Verteilungsmuster verglichen wird, die Unterschiede zwischen den Druckkraft-Verteilungsmustern in Steuersignale zum Steuern einer Vorrichtung zum Herstellen von Einlagen derart umgesetzt werden, daß sich bei Benutzung der hergestellten Einlagen das Soll-Druckkraft-Verteilungsmuster ergibt.

4. Verfahren nach einem der vorhergehenden Ansprüche,
**dadurch gekennzeichnet**,
daß mindestens an den Orten auf der Meßanordnung oder Meßmatte, über denen ein Abschnitt des Körperteils oder Fußes schwebt bzw. nicht aufliegt, mittels zusätzlicher Distanzfühler die Abstände zwischen Körperteil und Oberfläche der Meßanordnung dreidimensional abgetastet und dem Rechner übermittelt werden, daß das Distanzmuster mit einem gespeicherten Soll-Distanzmuster im Rechner verglichen wird und daß die errechneten Steuersignale im Sinne einer Kompensation von Fehlformen modifiziert werden.

5. Vorrichtung zur Durchführung des Verfahrens nach einem der vorhergehenden Ansprüche, umfassend eine elektronische Meßanordnung (2) zur zwei- oder dreidimensionalen Messung der auf die Meßanordnung wirkenden Kräfte, die mit einem Rechner (3) verbunden ist, der Speichermittel zum Speichern von zwei- oder dreidimensionalen Druckkraftverteilungsmustern aufweist und der so ausgebildet ist, daß in einer dynamischen Messung Ausgangssignale entsprechend einem über eine Vielzahl von Messungen gemittelten Druckkraft-Verteilungsmuster unter Zuordnung eines momentan gemessenen Druckkraft-Verteilungsmusters zum zuvor gemessenen Druckkraft-Verteilungsmuster hinsichtlich der Lage in der Meßebene erzeugt werden und, daß die Unterschiede zwischen den gemessenen und den gespeicherten Druckkraft-Verteilungsmustern in Steuersignale umgesetzt werden, die über Datenübertragungsmittel einer Vorrichtung (4) zum Herstellen von Einlagen oder dergleichen zugeführt werden.

6. Vorrichtung nach Anspruch 5,
**dadurch gekennzeichnet**,
daß auf der Meßanordnung (2) eine Vielzahl von Distanzfühlern (6, 16) angeordnet ist, die derart ausgebildet und mit dem Rechner (3) verbunden sind, daß die Abstände zwischen einem Körperteil und der Oberfläche der Meßanordnung (2) dreidimensional abgetastet und dem Rechner (3) übermittelt werden, wobei der Rechner (3) derart ausgebildet ist, daß das übermittelte Distanzmuster mit einem gespeicherten Distanzmuster eines Sollwert-Satzes für das Körperteil verglichen und die errechneten Steuersignale im Sinne einer Kompensation von Fehlformen modifiziert werden.

7. Vorrichtung nach einem der Ansprüche 5 oder 6,
**dadurch gekennzeichnet**,
daß die Meßanordnung zur zwei- oder dreidimensionalen Messung der Druckkräfte eine Matrixanordnung von Kraftaufnehmern (7) umfaßt.

8. Vorrichtung nach einem der Ansprüche 5 bis 7,
**dadurch gekennzeichnet**,
daß die Distanzmeßfühler (6) im wesentlichen unmittelbar auf der Oberfläche der Meßanordnung (2) vorgesehen sind.

9. Vorrichtung nach einem der Ansprüche 5 bis 8,
**dadurch gekennzeichnet**,
daß die Distanzmeßfühler (6) als berührungslose Meßfühler ausgebildet sind.

10. Vorrichtung nach Anspruch 9,
**dadurch gekennzeichnet**,
daß die Distanzmeßfühler zur Entfernungsmessung als Kapazitäts- oder Induktivitätsfühler, vorzugsweise als Meßfühler zur Messung der Laufzeit reflektierter Licht- oder Ultraschallwellen ausgebildet sind.

11. Vorrichtung nach einem der Ansprüche 5 bis 10,
**dadurch gekennzeichnet**,
daß zur Herstellung von Einlagen, zwischen der Meßanordnung (2) und der Fußsohle eine Anordnung (8) zur Aufbringung definierter Kräfte auf die Fußsohlen-Bereiche vorgesehen ist, die mit der Oberfläche der Meßanordnung (2) nicht in Berührung stehen.

12. Vorrichtung nach Anspruch 11,
**dadurch gekennzeichnet**,
daß die Vorrichtung (8) zur Aufbringung von Druckkräften eine Folie (10) umfaßt, die auf der Oberfläche der Meßanordnung (2) dicht angebracht und über mindestens eine Leitung (15) mit einer druckerzeugenden Einheit (11) verbindbar ist, so daß bei Erzeugung von Druck in der druckerzeugenden Einheit (11) der Raum zwischen Fußsohle und Oberfläche der Meßanordnung einen fluidgefüllten Raum bildet und die Folie (10) an dem zu messenden Körperteil im wesentlichen vollständig aufliegt, und daß die Distanzmeßfühler (6) Ultraschallfühler sind und direkt in den fluidgefüllten Raum zwischen Folie (10) und ihrer Oberfläche abstrahlen.

13. Vorrichtung nach Anspruch 12,
**dadurch gekennzeichnet**,
daß die Ultraschall-Distanzmeßfühler (6) einen Matrixaufbau aus Piezofolie umfassen, deren Oberfläche über sich kreuzende Leiterbahnen kontaktiert und über eine multiplexe Anordnung angeregt und/oder abgefragt werde.

14. Vorrichtung nach einem der Ansprüche 5 bis 13,
**dadurch gekennzeichnet**,
daß die Vorrichtung (4) zum Herstellen von Einlagen oder dergleichen eine CNC-Fräse zum Herstellen ausgießbarer Formen und/oder Tiefziehformen umfaßt.

15. Vorrichtung nach einem der Ansprüche 5 bis 13,
**dadurch gekennzeichnet**,
daß die Vorrichtung (4) zum Herstellen von Einlagen oder dergleichen eine Kunststoff-Formmaschine umfaßt, der Steuersignale zur ortsabhängigen Einstellung der Materialsteifigkeit zuführbar sind.

16. Verwendung einer Vorrichtung nach einem der Ansprüche 5 bis 15 zur Herstellung von orthopädischen Einlagen oder Fußbetten.

17. Verwendung einer Vorrichtung nach einem der Ansprüche 5 bis 15 zur Herstellung der Sitz- und/oder Lehnenflächen von Sitzmöbeln, Kraftfahrzeugsitzen oder dergleichen.

18. Verwendung der Vorrichtung nach einem der Ansprüche 5 bis 15 zur Herstellung von Liegemöbeln.

19. Verwendung der Vorrichtung nach einem der Ansprüche 5 bis 15 zur Herstellung der Verbindungsflächen zwischen Prothesen bzw. Orthesen und den entsprechenden Gliedstümpfen.

20. Verwendung der Vorrichtung nach einem der Ansprüche 5 bis 15 zur Herstellung von Zahnprothesen, insbesondere zu deren Anpassung an die Soll-Bewegungsgeometrie des Kauapparates eines Patienten.

## Claims

1. Process for manufacturing inserts or similar articles, according to which a patient moving the part of his/her body to be adjusted (foot, buttocks/back, teeth) in a defined manner repeatedly walks/sits/lies/bites on/over an electronic measuring arrangement for two- or three-dimensional measurement of the forces acting on said measuring arrangement, whereby said electronic measuring arrangement generates, in respect of the position in the measuring plane and in the course of a dynamic measuring operation, output signals corresponding to a compressive-force distribution pattern averaged for a plurality of measurements while assigning a currently measured compressive-force distribution pattern to the previously measured compressive-force distribution pattern, said output signals being fed to a computer and compared with a stored nominal compressive-force distribution pattern, whereupon the differences between the compressive-force distribution patterns are converted into control signals for controlling a device for manufacturing inserts (surfaces of seating furniture, surfaces of furniture for lying on, dentures) in respect of the geometric dimensions of the inserts (surfaces of seating furniture, surfaces of furniture for lying on, dentures) in such a way as to achieve the nominal compressive-force distribution pattern when use is made of the manufacturing inserts or similar articles.

2. Process for manufacturing inserts or similar articles, according to which a patient moving the part of his/her body to be adjusted (foot, buttocks/back, teeth) in a defined manner repeatedly strides/sits/lies/bites on/over an electronic measuring arrangement for two- or three-dimensional measurement of the forces acting on said measuring arrangement, whereby said electronic measuring arrangement generates, in respect of the position in the measuring plane and in the course of a dynamic measuring operation, output signals corresponding to a compressive-force distribution pattern averaged for a plurality of measurements while assigning a currently measured compressive-force distribution pattern to the previously measured compressive-force distribution pattern, said output signals being fed to a computer and compared with a stored nominal compressive-force distribution pattern, whereupon the differences between the compressive-force distribution patterns are converted into control signals for controlling a device for manufacturing inserts (surfaces of seating furniture, surfaces of furniture for lying on, dentures) in respect of the hardness or stiffness of the insert material (surfaces of seating furniture, surfaces of furniture for lying on, dentures) in such a way as to achieve the nominal compressive-force distribution pattern when use is made of the manufactured inserts or similar articles.

3. Process for manufacturing inserts, according to which a flexible measuring mat forming part of an electronic measuring arrangement is inserted into a shoe and the compressive-force distribution pattern acting upon the measuring mat as the patient walks or runs is averaged for a plurality of measured values and fed to a computer, whereby the averaging process is effected while assigning a currently measured compressive-force distribution pattern to the previously measured compressive-force distribution pattern in respect of its position in the measuring plane and compared with a stored nominal compressive-force distribution pattern, whereupon the differences between the compressive-force distribution patterns are converted into control signals for controlling a device for manufacturing inserts in such a way as to achieve the nominal compressive-force distribution pattern when use is made of the manufactured inserts.

4. Process according to one of the preceding claims,
**characterised in that**
the distances between the part of the body and the surface of the measuring arrangement are scanned three-dimensionally with the aid of additional distance sensors and transmitted to the computer, at least at those points of the measuring arrangement or measuring mat above which there is a gap between a section of said part of the body or foot or where there is no contact, in that the distance pattern is compared with a stored nominal distance pattern in the computer and in that the calculated control signals are modified so as to compensate for faulty shapes.

5. Device for implementing the process according to one of the preceding claims, comprising an electronic measuring arrangement (2) for two- or three-dimensional measurement of the forces acting on the measuring arrangement, said measuring arrangement being associated with a computer (3) equipped with storage means for storing two- or three-dimensional compressive-force distribution patterns and designed in such a way as to ensure that in the course of a dynamic measuring operation output signals are generated in accordance with a compressive-force distribution pattern corresponding to the average of a plurality of measuring results while assigning a currently measured compressive-force distribution pattern to the previously measured compressive-force distribution pattern in respect of the position in the measuring plane, and that the differences between the measured and the stored compressive-force distribution patterns are converted into control signals which are fed, via data transmitting means, to a device (4) for manufacturing inserts or similar articles.

6. Device according to Claim 5,
**characterised in that**
a plurality of distance sensors (6, 16) is disposed on the measuring arrangement (2), said distance sensors being so designed and associated with the computer (3) as to ensure that the distances between a part of the body and the surface of the measuring arrangement (2) are scanned three-dimensionally and transmitted to the computer (3), whereby the computer (3) is designed in such a way that the transmitted distance pattern is compared with a stored distance pattern of a set of nominal values for the part of the body in question and the calculated control signals are modified so as to compensate for faulty shapes.

7. Device according to one of the Claims 5 or 6,
**characterised in that**
the measuring arrangement for two- or three-dimensional measurement of the pressure forces comprises a matrix arrangement of force transducers (7).

8. Device according to one of Claims 5 to 7,
**characterised in that**
the distance sensors (6) are substantially provided directly on the surface of the measuring arrangement (2).

9. Device according to one of Claims 5 to 8,
**characterised in that**
the distance sensors (6) are designed as non-contact sensors.

10. Device according to Claim 9,
**characterised in that**
the distance sensors for measuring distances are designed as capacitive or inductive sensors, preferably as sensors for measuring the transit time of reflected light waves or ultrasonic waves.

11. Device according to one of Claims 5 to 10,
**characterised in that**
with a view to producing inserts an arrangement (8) for applying defined forces to those areas of the sole of the foot which are not in contact with the surface of the measuring arrangement (2) are provided between the measuring arrangement (2) and the sole of the foot.

12. Device according to Claim 11,
**characterised in that**
the device (8) for applying pressure forces comprises a foil which is tightly mounted on the surface of the measuring arrangement (2) and capable of being connected by means of at least one conduit (15) with a pressure-generating unit (11) so that whenever pressure is generated in the pressure-generating unit (11) the space between the sole of the foot and the surface of the measuring arrangement constitutes a space filled with fluid and the foil (10) is substantially in full contact with the part of the body to be measured, and in that the distance sensors (6) are ultrasound sensors and radiate directly into the fluid-filled space between foil (10) and their surface.

13. Device according to Claim 12,
**characterised in that**
the ultrasound distance sensors (6) comprise a matrix structure constituted by piezo-foil, the surface of which is contacted via intersecting conductor paths and excited and/or interrogated via a multiplex arrangement.

14. Device according to one of Claims 5 to 13,
**characterised in that**
the device (4) for manufacturing inserts or similar articles comprises a CNC milling machine for producing moulds suitable for casting and/or deep-drawing moulds.

15. Device according to one of Claims 5 to 13,
**characterised in that**
the device (4) for manufacturing inserts or similar articles comprises a machine for moulding plastics to which control signals for location-dependent setting of the material stiffness can be fed.

16. Use of a device according to one of Claims 5 to 15 for manufacturing orthopaedic inserts or orthopaedic socks.

17. Use of a device according to one of Claims 5 to 15 for manufacturing the seat surfaces and/or support surfaces of items of seating furniture, motor-vehicle seats or similar articles.

18. Use of the device according to one of Claims 5 to 15 for manufacturing items of furniture for lying on.

19. Use of the device according to one of Claims 5 to 15 for producing the joint surfaces between artificial limbs or limb replacements and the corresponding stumps.

20. Use of the device according to one of Claims 5 to 15 for manufacturing dentures, in particular for their adaptation to the nominal motional geometry of a patient's masticatory apparatus.

## Revendications

1. Procédé de fabrication de garnitures ou autres, un patient marchant/s'asseyant/se couchant/mordant plusieurs fois en un mouvement défini, par la partie du corps à adapter (pied, séant/dos, dents), sur un montage de mesure électronique, pour la mesure à deux ou à trois dimensions des forces exercées sur le montage de mesure, procédé dans lequel le montage de mesure électronique, dans une mesure dynamique, génère des signaux de sortie en conformité avec un modèle de répartition de pression moyenné sur une multiplicité de mesures, par association d'un modèle de répartition de pression mesuré instantanément à un modèle de répartition de pression mesuré préalablement, quant à la position dans le plan de mesure, ces signaux étant transmis à un ordinateur et comparés à un modèle de répartition de pression de consigne mémorisé, les différences entre les modèles de répartition de pression sont converties en signaux de commande pour piloter un dispositif de fabrication de garnitures (surfaces de sièges, surfaces de lits, prothèses dentaires), quant aux dimensions géométriques des garnitures (surfaces de sièges, surfaces de lits, prothèses dentaires), de sorte qu'il en résulte le modèle de répartition de pression de consigne lors de l'utilisation des garnitures fabriquées.

2. Procédé de fabrication de garnitures ou autres, un patient marchant/s'asseyant/se couchant/mordant plusieurs fois en un mouvement défini, par la partie du corps à adapter (pied, séant/dos, dents), sur un montage de mesure électronique, pour la mesure à deux ou à trois dimensions des forces exercées sur le montage de mesure, procédé dans lequel le montage de mesure électronique, dans une mesure dynamique, génère des signaux de sortie en conformité avec un modèle de répartition de pression moyenné sur une multiplicité de mesures, par association d'un modèle de répartition de pression mesuré instantanément à un modèle de répartition de pression mesuré préalablement, quant à la position dans le plan de mesure, ces signaux étant transmis à un ordinateur et comparés à un modèle de répartition de pression de consigne mémorisé, les différences entre les modèles de répartition de pression sont converties en signaux de commande pour piloter un dispositif de fabrication de garnitures (surfaces de sièges, surfaces de lits, prothèses dentaires), quant à la dureté ou à la rigidité du matériau (surfaces de sièges, surfaces de lits, prothèses dentaires), de sorte qu'il en résulte le modèle de répartition de pression de consigne lors de l'utilisation des garnitures fabriquées, ou autres.

3. Procédé de fabrication de garnitures, un mat de mesure flexible, en tant que partie d'un montage de mesure électronique, étant inséré dans une chaussure, le modèle de répartition de pression exercé sur le mat de mesure lors de la marche ou de la course du patient étant moyenné sur une multiplicité de mesures et transmis à un ordinateur, la moyenne étant produite par association d'un modèle de répartition de pression mesuré instantanément à un modèle de répartition de pression mesuré préalablement, quant à la position dans le plan de mesure, et le modèle moyenné étant comparé à un modèle de répartition de pression de consigne mémorisé, procédé dans lequel les différences entre les modèles de répartition de pression sont converties en signaux de commande pour piloter un dispositif de fabrication de garnitures, de sorte qu'il en résulte le modèle de répartition de pression de consigne lors de l'utilisation des garnitures fabriquées.

4. Procédé suivant l'une quelconque des revendications précédentes, caractérisé en ce que, aux endroits au moins du montage de mesure ou du mat de mesure, sur lequels une section de la partie du corps ou du pied plane et/ou ne s'applique pas, les distances entre la partie du corps et la surface du montage de mesure sont explorées en trois dimensions au moyen de capteurs d'écartement supplémentaires et transmises à l'ordinateur, en ce que le modèle d'écartement est comparé dans l'ordinateur à un modèle d'écartement de consigne mémorisé, et en ce que les signaux de commande calculés sont modifiés dans le sens d'une compensation des défauts de forme.

5. Dispositif pour la réalisation du procédé suivant l'une quelconque des revendications précédentes, comportant un montage de mesure électronique (2) pour la mesure à deux ou à trois dimensions des forces exercées sur le montage de mesure, ce montage étant relié à un ordinateur (3) qui présente des moyens de mémorisation pour mémoriser des modèles de répartition de pression à deux ou à trois dimensions et a une réalisation telle, que des signaux de sortie sont générés dans une mesure dynamique en conformité avec un modèle de répartition de pression moyenné sur une multiplicité de mesures, par association d'un modèle de répartition de pression mesuré instantanément à un modèle de répartition de pression mesuré préalablement, quant à la position dans le plan de mesure, et que les différences entre les modèles de répartition de pression mesurés et mémorisés sont converties en signaux de commande, qui sont transmis par des moyens de transmission de données à un dispositif (4) de fabrication de garnitures, ou autres.

6. Dispositif suivant la revendication 5, caractérisé en ce qu'une multiplicité de capteurs d'écartement (6, 16) est disposée sur le montage de mesure (2), ces capteurs étant réalisés et reliés à l'ordinateur (3), de sorte que les distances entre une partie du corps et la surface du montage de mesure (2) sont explorées en trois dimensions et transmises à l'ordinateur (3), l'ordinateur (3) ayant une réalisation telle que le modèle d'écartement moyenné est comparé à un modèle d'écartement mémorisé d'un jeu de valeurs de consigne pour la partie du corps, et que les signaux de commande calculés sont modifiés dans le sens d'une compensation des défauts de forme.

7. Dispositif suivant l'une quelconque des revendications 5 ou 6, caractérisé en ce que le montage de mesure à deux ou à trois dimensions des forces de pression comporte un agencement matriciel de capteurs de force (7).

8. Dispositif suivant l'une quelconque des revendications 5 à 7, caractérisé en ce que les capteurs d'écartement (6) sont essentiellement prévus directement sur la surface du montage de mesure (2).

9. Dispositif suivant l'une quelconque des revendication 5 à 8, caractérisé en ce que les capteurs d'écartement (6) sont réalisés sous forme de capteurs de proximité.

10. Dispositif suivant la revendication 9, caractérisé en ce que les capteurs pour la mesure d'écartement sont réalisés sous forme de capteurs capacitifs ou inductifs, sous forme de capteurs pour la mesure du temps de propagation d'ondes lumineuses ou ultrasonores réfléchies, de préférence.

11. Dispositif suivant l'une quelconque des revendications 5 à 10, caractérisé en ce que, pour la fabrication de garnitures, un montage (8) est prévu entre le montage de mesure (2) et la plante du pied pour appliquer des forces définies sur les zones de la plante du pied, qui ne sont pas en contact avec la surface du montage de mesure (2).

12. Dispositif suivant la revendication 11, caractérisé en ce que le dispositif (8) pour appliquer des forces de pression comporte un film (10), qui est appliqué au voisinage immédiat de la surface du montage de mesure (2) et peut être relié, par l'intermédiaire d'une conduite (15) au moins, à une unité génératrice de pression (11), de sorte que, lorsqu'une pression est générée dans l'unité (11), l'espace entre la plante du pied et la surface du montage de mesure forme un espace rempli de fluide, et que le film (10) s'applique intégralement sur la partie du corps à mesurer, et en ce que les capteurs d'écartement (6) sont des capteurs ultrasonores et émettent directement dans l'espace rempli de fluide entre le film (10) et la surface de ce dernier.

13. Dispositif suivant la revendication 12, caractérisé en ce que les capteurs d'écartement ultrasonores (6) comportent une structure matricielle de film piézo-électrique, dont la surface est mise en contact par l'intermédiaire de pistes conductives en croix, et est attaquée et/ou interrogée par un montage multiplex.

14. Dispositif suivant l'une quelconque des revendications 5 à 13, caractérisé en ce que le dispositif (4) pour la fabrication de garnitures, ou autres, comporte une fraiseuse CNC pour fabriquer des formes coulables et/ou embouties.

15. Dispositif suivant l'une quelconque des revendications 5 à 13, caractérisé en ce que le dispositif (4) pour la fabrication de garnitures, ou autres, comporte une machine de moulage de matière plastique, à laquelle peuvent être transmis des signaux de commande pour un réglage local de la rigidité du matériau.

16. Utilisation d'un dispositif suivant l'une quelconque des revendications 5 à 15 pour la fabrication de semelles orthopédiques ou de semelles intérieures.

17. Utilisation d'un dispositif suivant l'une quelconque des revendications 5 à 15 pour la fabrication des surfaces de sièges et/ou dossiers de sièges, sièges de véhicules automobiles, ou autres.

18. Utilisation du dispositif suivant l'une quelconque des revendications 5 à 15 pour la fabrication de lits.

19. Utilisation du dispositif suivant l'une quelconque des revendications 5 à 15 pour la fabrication des surfaces de jonction entre des prothèses et/ou orthèses et les membres correspondants.

20. Utilisation du dispositif suivant l'une quelconque des revendications 5 à 15 pour la fabrication de prothèses dentaires, pour l'adaptation de ces dernières à la géométrie de consigne du mouvement de l'appareil masticateur d'un patient, notamment.
